# EUROPEAN PATENT SPECIFICATION

(11) **EP 1 713 524 B1**
(45) Date of publication and mention of the grant of the patent: **13.04.2011**
(21) Application number: 05700087.9
(22) Date of filing: 19.01.2005
(51) Int. Cl.: A61L 27/30, A61L 27/50, A61L 27/04, A61F 2/00

(54) **BIOACTIVE COATING OF BIOMEDICAL IMPLANTS**
BIOAKTIVE BESCHICHTUNG VON BIOMEDIZINISCHEN IMPLANTATEN
REVETEMENT BIOACTIF POUR IMPLANTS BIOMEDICAUX

(30) Priority: 19.01.2004 AU 2004900202
(43) Date of publication of application: 25.10.2006
(73) Proprietor: UNIVERSITY OF SOUTH AUSTRALIA, Adelaide, S.A. 5000 (AU)
(72) Inventor: KUMAR, Sunil, Golden Grove, South Australia 5125 (AU); ZHANG, Hailong, Clayton, Victoria 3168 (AU); SIMPSON, Darren John, Gwanak-gu, Seoul 151-050 (KR); SMART, Roger, St.Clair, Tennyson, South Australia 5022 (AU)
(74) Representative: Kudlek & Grunert Patentanwälte
(86) International application number: PCT/AU2005/000055
(87) International publication number: WO 2005/067992

(56) References cited:
- EP-A1- 1 270 018
- EP-B1- 0 606 566
- WO-A1-00/32248
- WO-A1-95/04609
- WO-A2-03/075790
- US-A- 5 080 924
- US-A- 5 236 563
- US-A- 5 260 093
- US-A- 5 451 428
- US-A- 5 543 019
- US-A- 6 033 582
- US-A- 6 071 335
- NICOLAZO F ET AL: "Study of oxygen/TEOS plasmas and thin SiOx films obtained in an helicon diffusion reactor" SURFACE AND COATINGS TECHNOLOGY, vol. 98, 1998, pages 1578-1583, XP002482011
- KAWASHIMA N. ET AL: 'Comparison of Bioceramic Coatings on Metals from Sol-Gel, PACVD and Precipitation Processes.' KEY ENGINEERING MATERIALS vol. 253, 2003, pages 73 - 88, XP008091984
- STEVESON M. ET AL: 'XPS studies of low-temperature plasma graded oxide-silicate-silica layers on titanium.' SURFACE AND INTERFACE ANALYSIS. vol. 26, 1998, pages 1027 - 1034, XP008096387
- H-I YOO AND S-J KANG: 'Ceramics Interfaces', 2001 article SMART R. ET AL: 'New Approaches to Metal Ceramic and Bioceramic Interfacial Bonding.', XP008091985
- JACOBS P. AND KOWATSH R. ET AL: 'Sterrad Sterilization System: A new Technology for Instrument Sterilization.' END. SURG. vol. 1, 1993, pages 57 - 58, XP008096320

## Description

### FIELD OF THE INVENTION

The present invention relates to methods that can be used to modify the surfaces of a metallic biomedical implant such as an artificial bone, artificial joint or an artificial tooth root used in reconstructive surgery, to accelerate or improve *in situ* fixation of the implant to bone. The invention also relates to biomedical implants that are produced using the methods of the invention.

### BACKGROUND OF THE INVENTION

Medical and dental implants ('biomedical implants') such as hip, knee, dental and other bone implants are widely used in restorative surgery. For example, artificial joint implants are widely used in the field of orthopedics and artificial tooth root implants are attracting much attention in the field of dentistry. The implants are fixed in bone either by bonding to the bone by means of a suitable adhesive resin or by making use of the regrowth of a patient's bone tissue to fix the implant to the existing bone structure. The latter method is often referred to as 'intraosseous fixation' of the implant.

In most cases an implant is required to have high strength and for this reason they are typically made of metallic biomaterials such as titanium, titanium alloys, surgical stainless steel or cobalt-chromium. However, metallic substrates tend to provide a surface that is not very conducive to fixation through bone regrowth.

To assist in the regrowth and attachment of bone tissue to a metallic implant it is possible to apply a 'bioactive coating' to the implant. For example some bioactive materials such as many silica-containing glasses and glass-ceramics have been found to bond to living bone through a bone-like calcium hydroxyl phosphate (hydroxyapatite) layer in the body environment. For this reason many metallic implants that are used today have a hydroxyapatite coating that assists in the in situ deposition and growth of bone materials and hence the intraosseous fixation of the implant.

Kawashima et al., in "Comparison of Bioceramic Coatings on Metals from Sol-Gel, PACVD and Precipitation Processes", Key Eng. Mater. 253 (2003) 73, disclose treating the surface of an implant substrate with H₂O plasma prior to deposition of a silica layer. Three stages of plasma reaction are used, namely: surface cleaning using argon plasma; controlled surface oxidation of the metal using H₂O plasma; and controlled silicate reaction using a H₂O/TEOS combination.

In "Study of oxygen/TEOS plasmas and thin SiOx films obtained in an helicon diffusion reactor", Surf. Coat. Tech. 98 (1998) 1578, Nicolazo *et al.* disclose the deposition of thin films of silicon dioxide on Si substrates at ambient temperature in a O₂/TEOS (tetraethoxysilane) helicon diffusion plasma. Plasma composition and film properties are studied as functions of the TEOS to oxygen flow rate ratio, which is varied at fixed total gas flow rate.

However, it is relatively common for the hydoxyapatite coating to detach from the metallic implant due in part to the differential expansion and contraction experienced by implants in vivo. This ultimately leads to implant failure and a need for the patient to undergo revision surgery.

There is therefore a need to provide a method of forming a bioactive coated metallic biomedical implant that overcomes or at least reduces problems with the aforementioned implants.

### SUMMARY OF THE INVENTION

The present invention provides a method of forming a bioactive coating on a biomedical implant and to a corresponding biomedical implant as specified in the independent claims.

The method of the present invention provides an improved fixation of the implant relative to an uncoated implant or a known coated implant. Whether or not the fixation of an implant is"improved"relative to another implant can be determined in a number of ways, including but not limited to, in vivo studies after implantation of an implant treated according to the method of the present invention, or in vitro studies including studying the deposition of hydroxyapatite after treatment with simulated body fluid (SBF) or by physical testing such as shear testing. Suitable testing methods are described later in this specification.

The present invention includes the step of contacting at least part of the surface of the implant prior to implantation with a plasma gas containing a reactive hydroxylating oxidant species.

The reactive hydroxylating oxidant species that is included in the plasma gas is preferably hydrogen peroxide. The implant is also preferably stainless steel.

The step of contacting the surface of the implant prior to implantation with a plasma gas containing a reactive hydroxylating oxidant species is preceded by a step of exposing the implant to an organosilane or organosilicate species to form a silica coating on at least part of the implant prior to contact with the oxidant species.

The step of exposing the implant to an organosilane or organosilicate species to form a silica coating includes exposing the implant to a plasma gas formed from the organosilane or organosilicate. Preferably, the organosilane is tetraethoxysilane (TEOS).

Advantageously, it has been found that by exposing the implant to a plasma gas containing an organosilane or organosilicate it is possible to form a graded composite silica coating on the substrate that has improved performance, mechanical strength and adhesion. The improved properties are attributed to the formation of a functionally graded, nano-composite interfacial layer across the metal/metal oxide/metal silicate/silica transition with nanoparticles of each phase embedded in adjacent layers.

Following the methods of the present invention it is also possible to vary the physical properties of the silica coating by altering the ratio of organosilane to air in the plasma treatment step. Type I silica coatings can be obtained using a high organosilane/air ratio in the plasma, whereas Type II silica coatings can be obtained using a low organosilane/air ratio in the plasma. In a preferred form of the invention the silica coating is a Type II coating.

Using the methods of the present invention it is possible to form a relatively thin bioactive coating on an implant. An advantage of the formation of relatively thin coatings is that fine implant structures, such as surgical screws, can be coated with a bioactive coating without affecting the surface structure of the implant. Thus, the present invention also provides a bioactive implant having a hydroxylated, graded composite silica coating wherein the coating has an average thickness of less than 300 nm, more preferably less than 200 nm and most preferably less than 100 nm.

Using the methods of the present invention with hydrogen peroxide as the reactive hydroxylating oxidant species it is also possible to form a higher density of silanol groups on the treated surface than with other known methods. It has previously been suggested that it is the presence of hydroxyl groups on the surface of the treated implant that induces calcium hydroxy phosphate nucleation and hence promotes bone regrowth and attachment to the surface. Therefore the present invention also provides a metal implant having a bioactive coating that has a surface concentration of hydroxyl (OH) groups that is about 3 times the surface OH group concentration of a coating that is formed using methods other than hydrogen peroxide plasma treatment.

The present invention also includes an implant that is produced by one or more of the methods of the present invention.

Various terms that will be used throughout this specification have meanings that will be well understood by a skilled addressee. However, for ease of reference, some of these terms will now be defined.

The term "fixation" as used throughout the specification in relation to the fixation of an implant to bone is to be understood to mean the functional bonding or attachment of at least part of an implant to the existing bone structure of a patient.

The term "bioactive" as used throughout the specification in relation to implants is to be understood to mean that the properties of the surface of the implant are such that, *in vivo,* hard tissue is able to grow and attach to the surface of the implant.

The term "biomedical implant" as used throughout the specification is to be understood to mean any device that can be implanted and fixed into the skeletal structure of an animal or human patient. Examples of biomedical implants include (but are not limited to) artificial knee joints, artificial elbows, artificial knuckles, artificial hips, artificial tooth roots, cranio-facial screws, orthopaedic screws as well as short term implants comprising both internal and external implant structure components that are used for the fixation of bone fractures.

The term "plasma gas" as used throughout the specification is to be understood to mean a gas (or cloud) of charged and neutral particles exhibiting collective behaviour which is formed by excitation of a source of gas or vapour. A plasma gas containing a reactive hydroxylating oxidant species contains many chemically active charged and neutral species which react with the surface of the implant substrate. Typically, plasma gases are formed in a plasma chamber wherein a substrate is placed into the chamber and the plasma gases are formed around the substrate using a suitable radiofrequency or microwave frequency, voltage and current.

The term *"in vivo"* as used throughout the specification is to be understood to mean within the animal or human body. Likewise, the term *"in vitro"* as used throughout the specification is to be understood to mean outside of the animal or human body.

### GENERAL DESCRIPTION OF THE INVENTION

Turning now to a description of the invention in more detail.

The present invention provides a method of improving the fixation of an implanted metallic biomedical implant, the method including the step of contacting at least part of the surface of the implant prior to implantation with a plasma gas containing a reactive hydroxylating oxidant species.

As discussed previously, the successful fixing of an implant *in vivo* is generally assisted by bone regrowth and attachment to the implant. A common problem with uncoated metallic implants is that the metal surface provides a relatively poor interface for attachment of bone or other tissue. Whilst coated metallic implants provide an interface that is more suitable for bone regrowth, there is frequently a propensity for the coating to erode or detach from the metal implant substrate.

The method of the present invention provides an improved fixation of the implant relative to an uncoated implant or a known coated implant. Whether or not the fixation of an implant is "improved" relative to another implant can be determined in a number of ways, including but not limited to, *in vivo* studies after implantation of an implant treated according to the method of the present invention, or *in vitro* studies including studying the deposition of hydroxyapatite after treatment with simulated body fluid (SBF) or by physical testing such as shear testing. Suitable testing methods are described later in this specification.

The reactive hydroxylating oxidant species that is included in the plasma gas may be any agent that is able to form hydroxyl groups on the surface of the implant. Preferred reactive hydroxylating oxidant species are hydrogen peroxide, water, oxygen/water or air/water. In one particularly preferred form of the invention, the reactive hydroxylating oxidant species is hydrogen peroxide.

The rate and/or extent of reaction of the plasma gas containing the reactive hydroxylating oxidant species and the implant substrate can be controlled by controlling one or more of the plasma feed composition, the composition of the implant substrate, gas pressure, plasma power, voltage and process time.

Preferably the implant is exposed to the plasma in a plasma chamber held at between 107 Pa (8 x 10⁻¹ Torr) to 1.3 Pa (1 x 10⁻² Torr), preferably about 80 Pa (6 x 10⁻¹ Torr). It will be appreciated that the range of suitable working pressures in the plasma chamber could be extended using high vacuum plasma chambers. Preferably the temperature at the substrate surface during the plasma treatment step is less than about 80 C.

In the method of the present invention the implant is held within the plasma chamber and is surrounded by plasma gas containing the reactive hydroxylating oxidant species. Advantageously, the plasma gas treatment is a non-line-of-sight treatment which means that complex three-dimensional implants can be effectively coated.

The metallic implant may be made from any suitable bioactive surgical grade metal, including (but not limited to) stainless steel, titanium, titanium alloys, cobalt-chromium and other related alloys.

In the present invention the step of contacting the surface of the implant prior to implantation with a plasma gas containing a reactive hydroxylating oxidant species is preceded by a step of exposing the implant to an organosilane or organosilicate species to form a composite silica coating on at least part of the implant prior to contact with the reactive hydroxylating oxidant species.

The step of exposing the implant to an organosilane or organosilicate species includes exposing the metallic implant to a plasma gas containing the organosilicate or organosilane species. The organosilane is preferably a tetraalkylorganosilane containing alkyl groups having between 1 and 10 carbon atoms, more preferably 1 to 5 carbon atoms. In practice it is found that during the plasma assisted composite silica coating deposition step, the silica is deposited onto the implant surface and the lower alkoxy group from the organosilane forms the corresponding lower alkyl alcohol as a by-product. It is most preferable that the by-product alcohol is volatile enough to be removed by evaporation during the deposition step so that the alcohol does not interfere with the deposition of the coating. For this reason, it is preferable that the alkyl group of the organosilane forms a corresponding alcohol having a boiling point that is low enough for the alcohol to evaporate during the plasma assisted deposition step. Using the deposition conditions described herein, tetrapropoxysilane, tetraethoxysilane (TEOS) or tetramethoxysilane (TMOS) are the preferred organosilanes. In a particularly preferred form of the invention the organosilane is tetraethoxysilane (TEOS).

It has been found that using the methods of the present invention it is possible to form a silica coating having a functionally graded composite structure on the implant surface. The composite structure comprises a metal oxide/silicate/silica structure which has been found to provide excellent mechanical properties. The composite nature and functional gradation (chemical and physical properties) of this coating have been described (Smart et al.: New Approaches to Metal Ceramic and Bioceramic Interfacial Bonding, in Ceramic Interfaces 2, eds H-I Yoo and S-J Kang, Inst. Materials Communications Ltd. (UK), 2001, pp 293 and Stevenson et al., Surf. Interface Anal. 26, 1027-1034 (1998)).

The present inventors have now also discovered that by altering the ratio of TEOS and air in the plasma gas it is possible to alter the surface structure and composition of the coating layer which in turn affects the chemical stability (particularly interfacial hydrolysis), mechanical strength (particularly adhesion) and bioactivity of the coating layer. According to the methods of the present invention, Type I silica coatings can be obtained using a high TEOS/air ratio in the plasma, whereas Type II silica coatings can be obtained using a low TEOS/air ratio in the plasma. Using the methods of the present invention the Type I coatings generally had a thickness of about 300 nm whilst Type II coatings generally had a thickness of about 100 nm.

In forming a Type II composite silica coating it has been found that it is important to allow enough time during the plasma assisted silica coating step to allow for the gas phase to remove any of the lower alkyl alcohol that is formed.

The present inventors have previously shown that plasma-deposited thin-film coatings of silica on metallic implants achieve improved performance, mechanical strength and adhesion (Smart et al.: New Approaches to Metal Ceramic and Bioceramic Interfacial Bonding, in Ceramic Interfaces 2, eds H-I Yoo and S-J Kang, Inst. Materials Communications Ltd. (UK), 2001, pp293 and Kawashima et al.: Comparison of Bioceramic Coatings on Metals from Sol-gel, PACVD and Precipitation Processes, in Ceramic Interfaces 3: Properties and Applications, ed. K Uematsu, Trans Tech Publications, 2003, in press). The reason for these improvements is the formation of a functionally graded, nano-composite interfacial layer across the metal/metal oxide/metal silicate/silica transition with nanoparticles of each phase embedded in adjacent layers. This reaction layer is formed by the plasma products, particularly SiO ions and radicals, during plasma assisted chemical vapour deposition (PACVD) of TEOS vapour. Using the methods of the present invention hydroxylation of the silica surface can be used to give bioactivity, combined with the high mechanical strength and/or improved performance in physiological and implant applications. Previous experiments with these PACVD coatings have shown that the formation of this graded, composite structure is dependent on the conditions of plasma deposition, particularly TEOS vapour/air ratio, rate of deposition (operation pressure) and plasma power.

By following the method of this preferred form of the invention it is possible to form a composite silica coating layer on the surface of the metal implant. The silica coated implant is subsequently exposed to the plasma gas containing the reactive hydroxylating oxidant species which leads to the formation of a hydroxylated silica surface.

It has previously been reported that silica gel produced by hydrolysis and polycondensation of tetraethoxysilane (TEOS) in aqueous solutions containing polyethylene glycol causes the formation of hydroxyapatite on hydrated silica surface as the gel is soaked in a simulated body fluid (SBF) (Li et al., J. Am. Ceram. Soc., 75 (1992) 2094). In that case the authors suggested that hydroxylated silica gel induces calcium hydroxy phosphate nucleation on its surface in SBF and that the hydroxlated silica gel surface is highly catalytic for calcium hydroxy phosphate formation. Without intending to be bound by any one particular theory, it is thought that in the physiological body fluid (pH 7.4), a negatively charged silanol surface initially attracts calcium ions. This is followed by electrostratic interaction and hydrogen bonding (OH-PO₄³-) that brings phosphate anionic groups adjacent to Ca²⁺ ions. Near the silica gel surface, the accumulation of calcium ions and phosphate anionic groups enhances a precipitation reaction. A sufficient concentration of acidic silanol groups at the surface then induces calcium hydroxy phosphate formation.

After exposure to plasma containing hydrogen peroxide and then simulated body fluid (SBF) at 37°C it was found that Type I coatings tended to be susceptible to hydrolysis and dissolution whereas Type II silica coatings were stable under the test conditions. Therefore in one preferred form of the invention the method includes the step of exposing the implant substrate to a plasma gas containing a low TEOS/air ratio.

It is desirable to remove surface impurities such as hydrocarbons from the surface of the implant prior to exposing the implant to a plasma gas containing either the reactive hydroxylating oxidant species or the organosilane species. The surface impurities may be removed using any suitable method, although preferably the surface impurities are removed by either exposing the implant to a plasma gas of dry air or alternatively by exposing the implant to an argon plasma gas to remove hydrocarbon impurities followed by a plasma gas containing water to reoxidise the surface.

An implant that is coated according to the methods of the present invention may be used directly for implantation into a patient. The hydroxylated coating may then assist in the formation of a hydroxyapatite layer *in vivo.* Alternatively, the implant may be exposed to simulated body fluid (SBF) prior to implantation. Such exposure may be used to form a hydroxyapatite layer *in vitro* prior to implantation.

The present invention also includes an implant that is produced by one or more of the methods of the present invention.

An advantage of the method of the present invention is that the step of contacting at least part of the surface of the implant (with or without a composite silica coating) with a plasma gas containing the reactive hydroxylating oxidant species is that it is possible to form a higher density of silanol groups on the treated surface than with other known methods. As discussed previously, it has previously been suggested that it is the presence of hydroxyl groups on the surface of the treated implant that induces calcium hydroxy phosphate nucleation and hence promotes bone regrowth and attachment to the surface. Therefore the present invention also provides a metal implant having a bioactive coating that has a surface concentration of hydroxyl (OH) groups that is about 3 times the surface OH group concentration of a coating that is formed using methods other than the methods of the present invention.

Quantification of the extent of hydroxylation of the surface of the implant can be achieved by treating the implant with trifluoroacetic anhydride so that the hydroxylated sites are fluorinated and subsequently measuring the fluorine content using a suitable method such as X-ray photoelectron spectroscopy. In practice it has been found that the surface of a cobalt-chromium implant that has been treated with a plasma gas containing water as the reactive hydroxylating oxidant species had about 6 at.% fluorine, whilst a cobalt-chromium implant that was treated with a plasma gas containing hydrogen peroxide as the reactive hydroxylating oxidant species had about 19 at.% fluorine.

### BRIEF DESCRIPTION OF THE FIGURES

Certain aspects of embodiments of the invention are shown in the accompanying figures, in which:
Figure 1 is a bar graph showing the measured contact angles for Ti and SS screws following various treatments. In the graph, AR = as received, PP = plasma processed, SP = solution processed and SPHT = solution processed/heat treated.
Figure 2 is a bar graph showing the atomic concentrations of treated and untreated stainless steel (SS) screws. In the graph, AR = as received, PP = plasma processed, SP = solution processed, SPHT = solution processed/heat treated, and HA = hydroxyapatite coated.
Figure 3 shows XPS curve fitted O1s regions of as untreated and treated stainless steel (SS) screws. In the graph, AR = as received, PP = plasma processed, SP = solution processed and SPHT = solution processed/heat treated.
Figure 4 is a bar graph showing atomic concentrations of untreated and treated stainless steel (SS) screws exposed to TFAA vapour. In the graph, AR = as received, PP = plasma processed, SP = solution processed and SPHT = solution processed/heat treated.
Figure 5 is a bar graph showing atomic concentrations of untreated and treated titanium (Ti) screws. In the graph, AR = as received, PP = plasma processed, SP = solution processed, SPHT = solution processed/heat treated, and HA = hydroxyapatite coated.
Figure 6 shows XPS curve fitted O1s regions of untreated and treated titanium (Ti) screws. In the graph, AR = as received, PP = plasma processed, SP = solution processed and SPHT = solution processed/heat treated.
Figure 7 is a bar graph showing atomic concentrations of untreated and treated titanium (Ti) screws exposed to TFAA vapour. In the graph, AR = as received, PP = plasma processed, SP = solution processed and SPHT = solution processed/heat treated.
Figure 8 is a loading-unloading curve of a plasma processed stainless steel screw head indented with a 5 µm tip at a maximum of 10mN.
Figure 9 is an infrared spectrum of a Type I hydroxylated silica coating.
Figure 10 is an XPS wide scan spectrum of a Type I hydroxylated silica coating.
Figure 11 is a deconvoluted C1s photoelectron spectrum of a Type I hydroxylated silica coating.
Figure 12 is an XPS wide scan spectrum of a Type I hydroxylated silica coating after immersion in SBF solution at 37°C for 14 days.
Figure 13 is an infrared spectrum of a Type II hydroxylated silica coating.
Figure 14 is an XPS wide scan spectrum of a Type II hydroxylated silica coating.
Figure 15 is a silicon chemical state plot showing the distribution o a number of silicon-oxygen species, indicating the modified Auger parameter values determined for Type I and Type II silica coatings.
Figure 16 is an XPS wide scan of a Type II hydroxylated silica coating after immersion in SBF with amine at 37°C for 14 days.
Figure 17 is an XPS wide scan of a Type II hydroxylated silica coating after immersion in SBF without amine solution at 37°C for 14 days.
Figure 18 is an infrared spectrum of calcium hydroxy phosphate deposited onto the type II silica coating after immersion in SBF (without amine) solution at 37°C for 14 days.
Figure 19 shows scanning electron micrographs showing surface morphologies of a Type II hydroxylated silica coating (a) as deposited and (b) after immersion in SBF without amine solution at 37°C for 14 days.
Figure 20 shows scanning electron micrographs showing surface morphologies of a Type II hydroxylated silica coating after immersion in 1.5SBF without amine solution at 37°C for 5 days at (a) low and (b) high magnification showing aggregated formed at higher super-saturation on top of the continuous layer.

### DESCRIPTION OF EMBODIMENTS OF THE INVENTION

Reference will now be made to examples that embody the above general principles of the present invention. However, it is to be understood that the following description is not to limit the generality of the above description.

### Example 1 - Treatment of implant substrates with plasma containing a reactive hydroxylating oxidant species

Commercially available titanium or stainless steel self-tapping cortex screws, 3.5mm diameter and 30 mm long were treated using the methods of the invention and were compared with untreated samples and also with samples that had been treated using published methods. Brief sample preparation details are:
- AR (As-Received) samples: titanium and stainless steel screws as-received, in polyethylene bags.
- PP (Plasma Processed) samples: Using the methods of the present invention, the samples were subjected to a two-step plasma treatment using H₂O₂ as the reactive hydroxylating oxidant species (5 min air plasma, followed by 5 min air/30% H₂O₂ plasma). The system used to prepare these samples was a Harrick radio frequency plasma unit (model PDC-32G). The flow rates of air were controlled by using two mass flow controllers (MFCs). The flow rate range of the MFCs was 3.34 x 10⁻⁸ - 1.67 x 10⁻⁶ m³/s (2-100 sccm) based on N₂ gas correction factor. Prior to placing the screws into the chamber, the headspace above the reservoir containing 30% H₂O₂ solution was evacuated. The H₂O₂ reservoir was maintained at room temperature in a water bath. Approximately 40 ml of 30% H₂O₂ was used that sat well above the level of the frit thereby allowing air to bubble through it. The maximum power setting (100 W) was used in both the steps: step 1 - air plasma treatment @ 1.67 x 10⁻⁷ m³/s (10sccm) for 5 min; and step 2 - air/30% HₛO₂ plasma treatment @ 3.34 x 10⁻⁸ m³/s (2sccm) for min.
- SP (Solution Processed) samples: 24 h treatment in 30% H₂O₂ solution kept at 60°C followed by rinsing thrice in Milli-Q water and then blown dry with compressed argon (see for example United States patent 5,855,612).
- SP-HT: SP sample was heat-treated at 400°C in air for 1 h, followed by cooling in the furnace (see for example Wang et al., Biomaterials 23 (2002) 1353.
- HA-AR, HA-PP, HA-SP and HA-SP-HT samples: soaking of the AR, PP, SP and SP-HT screws respectively in SBF kept at 37°C for 14 days with one replenishment on day 8. After 14 days of SBF treatment, the HA (calcium hydroxy phosphate) coated samples were rinsed thrice with Milli-Q water and then blow dry with compressed argon.

A total of 24 (12 titanium + 12 stainless steel) screws were used for the present study, inclusive of one repeat run (except for SP, SP-HT, HA-SP-HT). A greenish tinge of the titanium screws suggested that they were anodized by the manufacturer. The shape of the screws was such that almost all the measurements were performed on a single screw (each cut into two pieces). Both titanium and stainless steel screws were treated independently. SBF is the inorganic component of blood serum and was prepared using a literature method (Ohtsuki et al., J. Biomed. Mater. Res. 35 (1997) 39). Trishydroxymethylaminomethane was excluded from the SBF solution because we found that it was able to inhibit/reduce the growth of HA from SBF.

All of the treated samples, including the AR samples, were autoclaved before any measurements were performed. This was done as the autoclaving of implants is a regular procedure followed for implantation.

### 1.1 - Surface morphology of treated screws

The scanning electron microscopy (SEM) technique in its secondary electron imaging mode was used for investigating the surface morphology of the samples produced.

### Stainless steel cortex screws

The surfaces of AR-stainless steel screws were very smooth when observed by SEM. Fine ripples in the surface finish suggest that the manufacturer used electropolishing. The surface topography was not altered by 100W 30% H₂O₂ plasma processing (PP). No change in the surface topography of solution processed (SP) screw was observed following 24 hours immersion in highly oxidising H₂O₂ solution solution at 60°C. This combined with heat treatment at 400°C ((SPHT) sample) also had no effect on surface finish although there was some change in colouration (from metallic silver luster to golden lustre). Of all the stainless steel samples treated with simulated body fluid ('SBF') and subsequently examined by SEM, only the HA-PP screws exhibited colloidal calcium hydroxy phosphate particles on its surface. The SEM micrographs of the rest of the SBF-treated stainless steel samples (HA-AR, HA-SP and HA SP-HT) did not show the presence of such colloidal particles.

The calcium hydroxy phosphate particles observed on the HA-PP sample surface formed a continuous layer (as revealed by XPS).

### 1.2 - Contact Angle measurements (Sessile drop method)

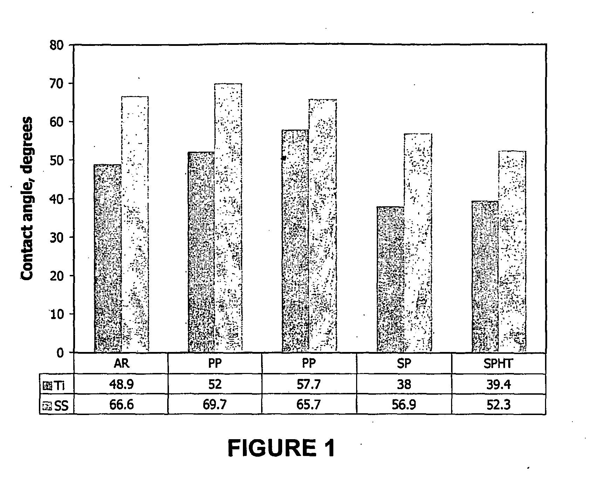

The wetting characteristics of the AR, plasma- and solution-treated samples were studied by making contact angle measurements. The objective of this study was to see if it was possible to differentiate and characterise differently treated surfaces on the basis of this simple measurement. Results from Sessile drop measurements of a water droplet sitting in the screw head well are summarized in Figure 1 for both stainless steel and titanium screws. The data in Figure 1 were obtained from images of silhouette cast by the water drop in the screw well. It must be acknowledged that the contact angle measurements may have been compromised due to the complex, non-ideal shape of the screw heads.

### Stainless steel cortex screws

Contact angles for the PP treated screws (66° and 70°) were virtually the same as the AR screw (67°). There is no doubt that the plasma processing improves surface reactivity for HA formation, but no change in contact angle can be measured after air-exposure delay and autoclaving prior to the measurements. Both the SP and SPHT screws displayed lower contact angles (57° and 52°, respectively).

### Titanium cortex screws

Contact angles for PP-titanium screws (52° and 58°) were slightly higher than the AR screw (49°). Both SP and SPHT screws displayed lower contact angles (38° and 39°), respectively, apparently due to the extensive reaction and gel formation.

### 1.3 - X-ray photoelectron spectroscopy (XPS) measurements

The XPS measurements were carried out for determining the chemical compositions of the AR and plasma and solution treated samples. The O1s photoelectron spectra of AR, PP, SP and SPHT samples, both stainless steel and titanium, were curve fitted for possible measurement of the degree of hydroxylation. Chemical derivatisation reaction with trifluoroacetic anhydride (TFAA) vapour was also employed for measuring the degree of hydroxylation of these surfaces (Dickie et al., Anal. Chem. 54 (1982) 2045). The total fluorine content on the derivatised surface, a direct measure of the surface hydroxylation, was determined by XPS.

### Stainless steel cortex screws

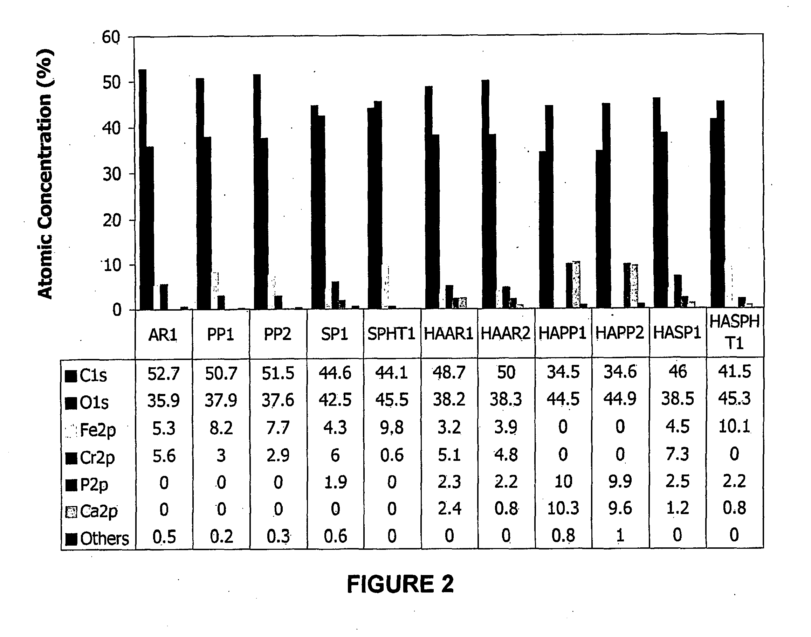

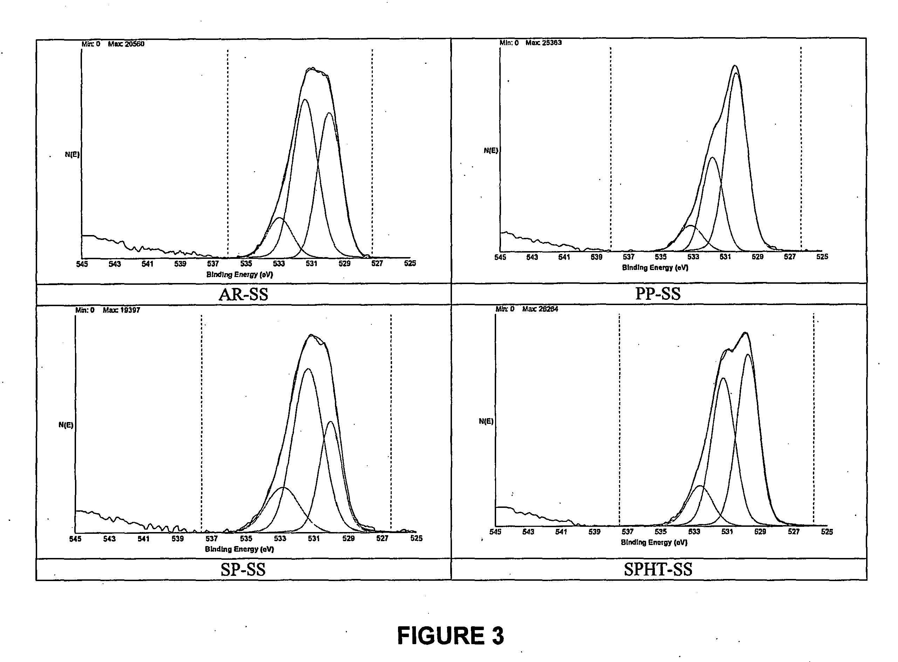

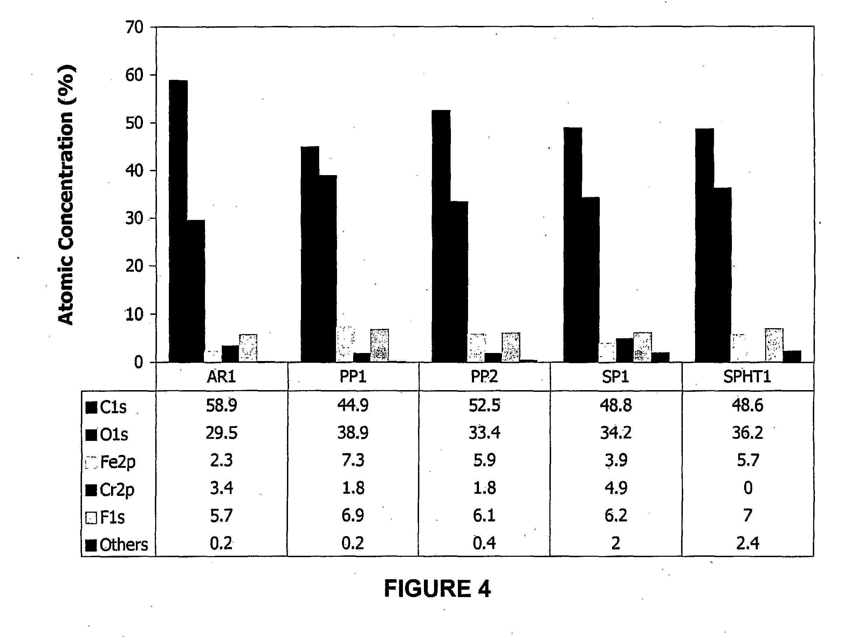

The XPS chemical compositions of the AR and treated stainless steel screws are summarised in Figure 2. The curve fitting of the O1s photoelectron peaks of various stainless steel samples, shown in Figure 3, was performed for obtaining different binding energy (B.E.) values that reflect various chemical (bonding) states of the oxygen atom in the samples. The B.E. values thus obtained are listed in Table 1. The interpretation of the curve fitted data is based on the reference B.E. values for the oxides and hydroxides of Fe and Cr listed in Table 2 (Wagner *et al.,* NIST X-ray Photoelectron Spectroscopy Database, NIST Standard Reference Database 20, Version 3.2 (Web version)). The XPS chemical compositions of AR and treated stainless steel screws exposed to the TFAA vapour are summarised in Figure 4.

From atomic concentration data in Figure 2, the 30% H₂O₂ plasma treatment did not change the C1s photoelectron contribution with respect to the AR screw. However, the Fe2p/Cr2p ratio increased significantly. There was a significant reduction in the C1s level following immersion in highly oxidizing H₂O₂ solution (SP) and a slight decrease in the Fe2p/Cr2p ratio. In contrast, the SPHT screw displayed the highest Fe2p/Cr2p ratio. As expected H₂O₂ solution increased the ratio of oxygen to iron and chromium. Ca2p and P2p photoelectron signals were detected on all screws immersed in SBF. However, only the PP screws were covered by a continuous calcium phosphate based layer. The Fe2p and Cr2p signals from these screws were completely attenuated. As mentioned, this finding, confirmed by a repeat experiment, suggests that the PP treated sample will have high bioactivity and therefore will be suitable to the fixation of stainless steel implants such as screws, external fixation pins, etc.

The hydroxyl ('OH') component (∼ 531.5 eV) in the O1s region for the PP screw (29.3%) was reduced with respect to the AR screw (48.1 %) (Table 1). A 20 min PP treatment further reduced the OH contribution to 24.7%. The OH contribution from the SP screw was quite high (55.2%) and indicative of a thin layer of gel. Heat treatment (SPHT) reduced this contribution slightly to 47.2%. The normal 5 min plasma processing (PP) reduced the amount of molecular water associated with the AR screw and there was further reduction with the longer 20 minute treatment. Heat treatment also reduced the amount of trapped water in the SP screw. Clearly, the heat treatment reduced the OH contribution but a higher temperature is required to remove it completely. The metallic component (∼707 eV) in the Fe2p region was clearly visible in the AR screw but disappeared following PP and SP treatments. Atomic concentration data of screws exposed to trifluoroacetic acid ('TFAA') vapour shows increases in surface hydroxylation (proportional to the fluorine content in Figure 4) as a result of plasma processing of stainless steel screws. However, the complex shape of the screws appears to affect the total fluorine detected per unit area, thereby making the quantification of plasma hydroxylation difficult.

### Titanium cortex screws

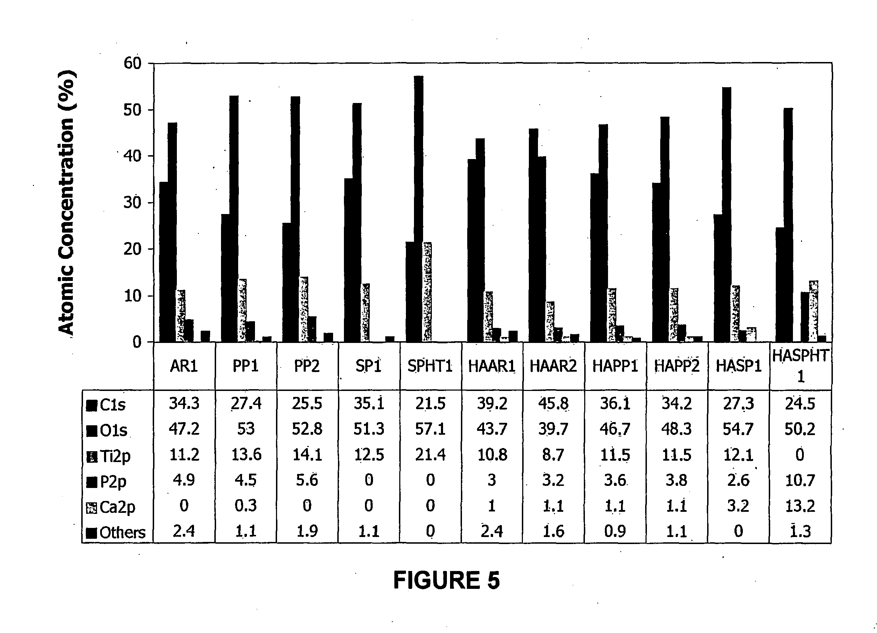

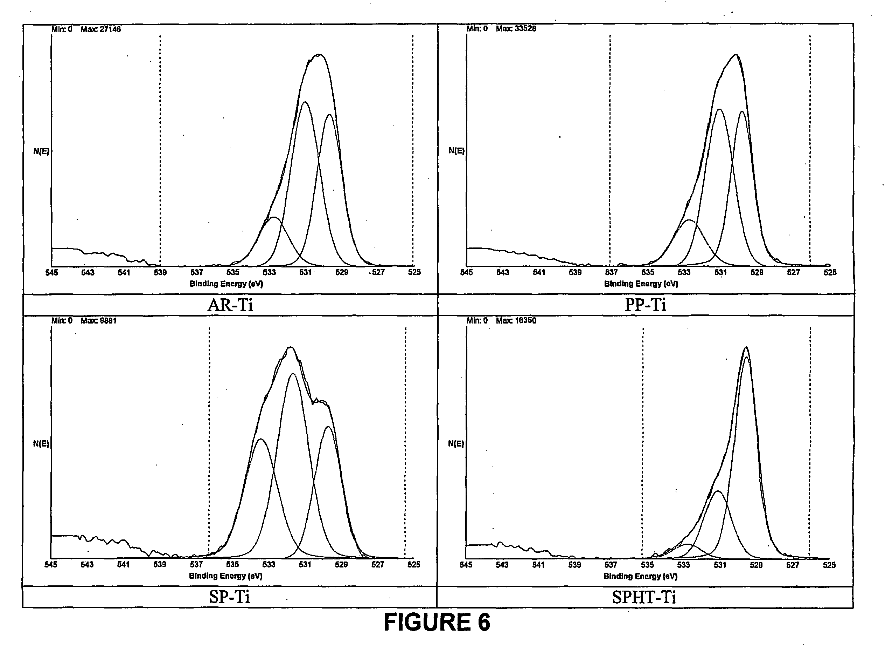

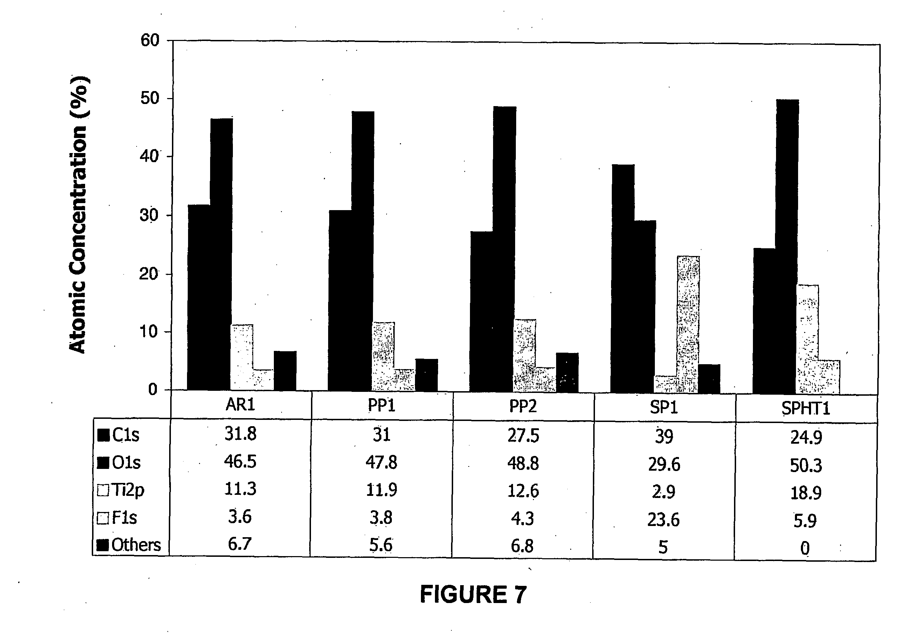

The XPS chemical compositions of the AR and treated titanium screws are summarized in Figure 5. The curve fitting of the O1s photoelectron peaks of various titanium samples, shown in Figure 6, was performed for obtaining different binding energy (B.E.) values that reflect various chemical (bonding) states of the oxygen atom in the samples. The B.E. values thus obtained are listed in Table 1. The interpretation of the curve fitted data is based on the reference B.E. values for the oxides and hydroxides of titanium listed in Table 2 (Wagner *et al*., NIST X-ray Photoelectron. Spectroscopy Database, NIST Standard Reference Database 20, Version 3.2 (Web version)). The XPS chemical compositions of AR and treated titanium screws exposed to the TFAA vapour are summarised in Figure 7.

The PP treatment reduced the C1s level with respect to the AR screw. There was no significant reduction in the C1s level following immersion in highly oxidizing H₂O₂ solution (SP). However, the SPHT screw displayed the lowest C1s level. The use of H₂O₂ with and without plasma did not significantly alter the O1s/Ti2p ratio. Surprisingly, this ratio decreased for the SPHT screw. Ca2p and P2p photoelectron signals were detected on all screws immersed in SBF. However, only the SPHT screw was covered by a continuous calcium hydroxy phosphate layer. The Ti2p photoelectron signal from this screw was completely attenuated. However, this sample exhibited quite rough and powdery/flaky/spalling surfaces which are unlikely to meet quality assurance standards for orthopaedic implantation in humans.

The OH component (531.2 eV) in the O1s region for the PP screw (40.9%) was virtually the same with respect to the AR screw (42.6%) (Table 1). However, a longer experiment was carried out to satisfy any doubts. A 20 min PP treatment reduced the OH contribution markedly to 28.9%. Contributions from OH (44.1 %) and water (30.5%) dominate the O1s spectrum of the SP screw. In contrast, the OH component was smaller for the SPHT screw (26.1 %). The normal 5 min plasma processing (PP) did not significantly reduce the amount of molecular water associated with the AR screw. However, there was marked reduction with the longer 20 minute treatment. Heat treatment also reduced the amount of trapped water in the SP screw. Atomic concentration data of screws exposed to TFAA vapour show slight increases in surface hydroxylation (proportional to the fluorine content of Figure 7) as a result of plasma and solution processing of titanium screws. However, as stated above for the stainless steel samples, the complex shape of the titanium screws appears to affect the total fluorine detected per unit area, thereby making the quantification of plasma hydroxylation difficult.

**Table 1. Binding energy positions and area contributions to 01 s peak in Figures 4 and 7.**

| Treatment | Stainless Steel | | Titanium | |
|---|---|---|---|---|
| | Position (eV) | Area (%) | Position (eV) | Area (%) |
| AR | 529.90 | 39.8 | 529.83 | 42.0 |
| | 531.37 | 48.1 | 531.15 | 42.6 |
| | 532.95 | 12.1 | 532.80 | 15.4 |
| PP | 530.32 | 61.4 | 529.90 | 44.1 |
| | 531.80 | 29.3 | 531.17 | 40.9 |
| | 533.16 | 9.3 | 532.80 | 15.0 |
| PP (20 min) | 529.89 | 71.4 | 530.09 | 67.2 |
| | 531.40 | 24.7 | 531.51 | 28.9 |
| | 532.89 | 3.9 | 532.81 | 3.9 |
| SP | 530.05 | 28.2 | 529.75 | 25.4 |
| | 531.37 | 55.2 | 531.69 | 44.1 |
| | 532.85 | 16.7 | 533.44 | 30.5 |
| SPHT | 529.73 | 42.6 | 529.60 | 67.9 |
| | 531.25 | 47.2 | 531.13 | 26.1 |
| | 532.81 | 10.2 | 532.80 | 6.0 |

**Table 2. Reference binding energy (BE) values for the O1s region**

| Substance | BE range (eV) |
|---|---|
| Fe oxide | 529.5 - 530.3 |
| Fe hydroxide | 530.1 - 531.8 |
| Cr oxide | 529.3 - 530.8 |
| Cr hydroxide | 530.8 - 531.4 |
| Titanium oxide | 529.6 - 530.1 |
| Titanium-OH (oxyhydroxide) | 531.1 - 531.7 |
| H₂O | 532.8 - 535.1 |

### 1.4 - Indentation and scratch testing

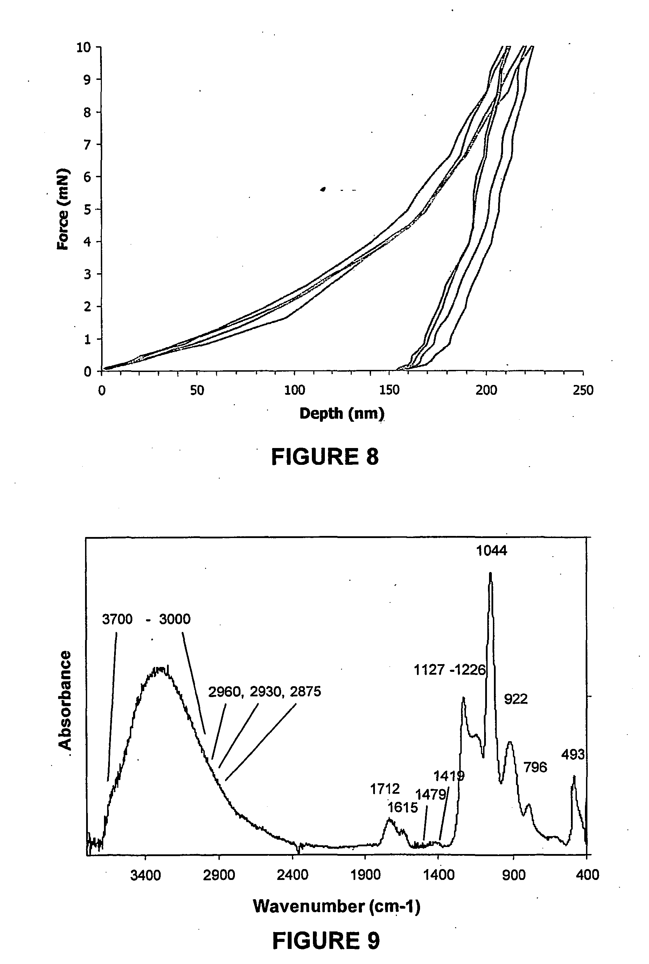

A microindenting machine (UMIS2000) was used in its indentation (hardness) and scratch testing modes for investigating the mechanical properties differences between plasma and solution processed screws. Owing to the curved surfaces of the screw head, all the scratch testing attempts failed. Therefore, only indentation results are presented below. A typical indentation (loading-unloading) curve obtained for a PP- stainless steel screw is shown in Figure 8. In the loading-unloading indentation mode, an indenter (5µm diameter tungsten carbide tip in our case) is used for applying an indenting load (force), which is incremented until the maximum load (10 mN in our case) is reached. It is then decremented in the same steps back to the contract force. The depth of penetration of the indenter, a function of the hardness of a material, is measured; the lower the penetration depth, the harder the material indented.

### Stainless steel cortex screws

Five indentations were performed on a screw head from each treatment. Apart from one or two erroneous results (out of five indentations), loading-unloading data on stainless steel screw heads were reproducible. The indentation curves obtained for the AR, PP and SP samples under a load of 10 mN are very similar (Figure 8 for a PP-Stainless steel sample, for example), resulting in -200 nm deep indentations. These results imply that the surface hardness of all these three types of samples is quite similar. Indentation values in the range 200-350 nm were observed for SPHT sample, implying relatively low hardness. The scatter in the data is very likely due to inherent roughness of the surfaces tested.

### Titanium cortex screws

Loading-unloading data on titanium screw heads were not reproducible. There is some indication from tests on AR and PP samples that 150-200 nm deep indentations were produced under a load of 10 mN. However, for the same applied load (10 mN) there was a large variation in the penetration depths obtained for the SP (200-800 nm) and SPHT (200- 2000 nm) samples, which very likely is a consequence of the rough titanium surfaces of these samples. Despite these variations, it was found that under the same applied load the overall penetration depths for the SP and SPHT samples were significantly higher (implying lower hardness values) than the AR and PP samples.

### Example 2 - Treatment of implant substrates with organosilanes follower by plasma treatment with a reactive hydoxylating oxidant species

Titanium sheet (99.7% purity) and tetraethoxysilane (TEOS) (98% purity) were purchased from Aldrich Chemical Co. and hydrogen peroxide (H₂O₂ 30% (Univar) analytical reagent was obtained from APS Finechem (Australia). The titanium substrates were prepared by cutting the 0.25mm thick sheet into 10 x 10 mm² pieces. Prior to plasma deposition, the metal pieces were rinsed in 5 wt.% KOH solution followed by a Milli-Q water wash, then immersed in a warm solution containing 1.6 wt.% HF, 33 wt% HNO₂ for 2-3 min followed by copious Milli-Q water rinse.

As mentioned above, the technique of plasma assisted chemical vapour deposition (PACVD) was employed for preparing thin-film coatings of hydroxylated silica onto titanium substrates. The configuration and use of a PACVD apparatus is known to a person skilled in the art. A radio frequency (13.56 MHz) plasma reactor (Harrick Scientific Corporation, model PDC-32G) operated at a power level of 40 W to 100 W was used for the deposition process. This plasma reactor and reaction conditions are normally used for removing hydrocarbon contamination from solid substrates such as windows or infrared spectroscopy cells. The pressure in the vacuum chamber was monitored by a pressure gauge (Edwards High Vaccum, model 8/1).

### 2.1 - PAVCD treatment of titanium surfaces

A small amount of TEOS (-2 ml) was placed in a glass bulb and around 40 ml of 30% H₂O₂ solution was placed in another glass bulb. Prior to placing the titanium substrates into the deposition chamber, the headspaces above the TEOS and H₂O₂ reservoirs were evacuated. Titanium substrates were then placed onto a glass plate located in the middle of the plasma chamber. The base pressure was about 3.9 Pa (3 x 10⁻² Torr). In the first plasma reaction step, dry air from a gas cylinder was introduced into the chamber at 1.67 x 10⁻⁷ m³/s (10 sccm) (standard cubic centimetre per minute) using a mass flow controller (MKS, model M100B) and the plasma reactor was activated for 20 min. This step removes surface impurities, particularly hydrocarbons, and the oxygen in air oxidizes the titanium surface. The chamber pressure was about 67 Pa (5 x 10⁻¹ Torr) during this pre-treatment step. In the second step that results in the formation of the silica coating, air was used as a carrier gas for transporting the TEOS vapour from its reservoir into the plasma chamber. The glass bulb containing TEOS was immersed in a water bath maintained at a constant temperature throughout this deposition step. This step lasted for 30 min at a pressure of about 67 Pa (5 x 10⁻¹ Torr). In order to vary the TEOS/air ratio, the TEOS flux transported into the plasma chamber was controlled by varying the temperature of the water bath containing the TEOS bulb, with the flow rate of air (the carrier gas) kept constant at 3.34 x 10⁻⁸ m³/s (2 sccm) and plus the 1.67 x 10⁻⁷ m³/s (10 sccm) of air from inlet port in the middle.

Two types of silica coatings were deposited. A Type I coating was obtained with a high TEOS/air ratio by maintaining the TEOS water bath temperature at 22 °C. A Type II coating was obtained with a low TEOS/air ratio from a water bath maintained at 8 C. The equilibrium vapour pressure of TEOS at 22 °C is 138 Pascals and at 8 °C it is 46 Pascals. Hence, the TEOS/air ratio in the Type

I silica coating is approximately three times greater than it is in the Type II coating.

In the third and final deposition step, the hydroxylation of the silica surface was carried out by treating it with the H₂O₂ plasma for 1 min. Air was used as a carrier gas for transporting the H₂O₂ vapour from the glass bulb into the plasma chamber. For this, air (maintained at 3.34 x 10⁻⁸ m³/s (2 sccm)) was bubbled through H₂O₂ solution, thereby producing a flow of H₂O₂ and water vapour. The chamber pressure was around 6x10⁻¹ Torr during this step. The temperature measured on the substrate during the deposition was was less than about 80°C and normally about 50°C to about 60°C. The thicknesses of Types I and II coatings estimated by SEM from cross sections were about 300 and 100 nm, respectively.

### 2.2 - In vitro testing of the bioactivity of treated surfaces

For studying the interaction of hydroxylated silica coatings with simulated body fluid (SBF), primarily aimed at growing calcium hydroxy phosphate on them, the SBF preparation method employed by Cho *et al.* was followed (Cho et al., J. Am. Ceram. Soc. 78 (1995) 1769). In this method, SBF is prepared by dissolving NaCl, NaHCO₃, KCI, K₂HPO₄.3H₂O, MgCl₂.6H₂O, CaCl₂ and Na2SO₄ reagents into Milli-Q water, and buffered at physiological pH 7.4 at 37°C with 50 mM trishydroxymethyl aminomethane [(CH₂OH)₃CNH₃] and 45 mM hydrochloric acid (HCl). As shown in Table 3, the ion concentration of SBF is nearly equal to that of human blood plasma. Both types of prepared silica coating surfaces were immersed in 30 ml of SBF solution in 50 ml polystyrene bottles and placed in an incubation room at 37°C. After the desired immersion time, the samples were removed from SBF and rinsed gently with Milli-Q water to remove salt residues prior to analysis.

**Table 3. Ion Concentrations of Simulated body fluid (SBF) and Human Blood Plasma**

| Ion Concentration (mM) | | | | | | | | |
|---|---|---|---|---|---|---|---|---|
| | Na⁺ | K⁺ | Ca²⁺ | Mg²⁺ | HCO₃⁻ | Cl⁻ | HPO₄²⁻ | SiO₄²⁻ |
| SBF | 142.0 | 5.0 | 2.5 | 1.5 | 4.2 | 147.8 | 1.0 | 0.5 |
| Human Plasma | 142.0 | 5.0 | 2.5 | 1.5 | 27.0 | 103.0 | 1.0 | 0.5 |

The treated titanium metal surfaces were analysed by X-ray photoelectron spectroscopy (XPS) using a 300W (15 kV and 20 mA) Mg Kα X-ray source (1253.6 eV). Wide-scan XPS spectra were recorded in the 0 to 1100 eV binding energy (BE) range at a pass energy of 93.9 eV. In order to get information about the chemical states of the elements present in the coatings, high-resolution XPS spectra (29.35 eV pass energy) of C 1s, Si 2p, and O1s were obtained. The modified Auger parameter was also used to obtain information on the chemical nature of silicon bonding in the coatings. Charge correction to the XPS binding energies was required due to the insulating nature of the coatings; a binding energy value of 284.8 eV for adventitious carbon was used for this purpose. A Fourier transform infrared (FTIR) spectrophotometer (Bio-Rad FTS-65) in its specular reflectance mode with a glancing angle of 30 degrees was used to obtain infrared vibrational spectra of the coatings, using 1024 scans at a resolution of 4 cm⁻¹. After background subtraction, the absorbance spectra were baseline corrected using the appropriate software. A scanning electron microscope (High-resolution, field emission CAMSCAN, model 2-90FE) was used to analyse the sample morphology.

As discussed previously, the presence of silanol (Si-OH) functional groups associated with negatively charged silica surfaces has been shown to provide a potent platform for growing calcium hydroxy phosphate (CHP) from SBF. In our experiments to increase silanol coverage on the coating, increased CHP growth was achieved using the peroxide plasma treatment. The following two types of hydroxylated silica coatings were prepared for studying the effect of TEOS/air ratio on their reaction, structural and compositional characteristics:
- Type I obtained using high TEOS/air ratio (TEOS containing glass bulb immersed in water bath at room temperature (22°C)); and
- Type II obtained using low TEOS/air ratio (TEOS containing glass bulb immersed in water bath at 8°C).

In assessing the potency of our hydroxylated silica coatings for the growth and adhesion of calcium hydroxy phosphate, the interaction of both Type I and Type II samples with SBF was investigated.

### 2.3 - Coatings deposited using high TEOS/air ratio (Type I coatings)

### 2.3.1 Infrared Studies

Figure 9 shows an infrared spectrum of a Type I coating, revealing absorption bands that are characteristics of hydroxylated silica. The strong and broad band between 3000 and 3700 cm⁻¹ in Figure 9 is associated with hydrogen bonded hydroxyl (O-H) and water species. The broad band centered at about 3300 cm⁻¹ is the bonded O-H stretch of the SiO-H structure and is accompanied by the v Si-O stretching vibration of the Si-OH species, centered at 922 cm⁻¹, within the coating (Iler, The Chemistry of Silica, John Wiley, 1979, p.634), indicating a substantial quantity of Si-OH present in the coating. Some fraction of the band at 3300 cm⁻¹ is due to the O-H stretches of water trapped in the coating. The small absorption band at 1615 cm⁻¹ is attributed to the bending vibration of water. Hence this Type I coating contains a very high concentration of SiOH groups and molecular water.

The infrared spectra of silica films are often assigned in the literature according to the calculations of Bell *et al.* (Bell et al., J. Phys. C 1 (1968) 299) and Bell and Dean (Bell and Dean, Discuss. Faraday Soc. 50 (1970) 55); the three regions around 1100, 800 and 450 cm⁻¹ are correlated, respectively, to stretching, bending and rocking modes of Si-O-Si bridges. The absorption bands observed at 1044, 796 and 493 cm⁻¹ are therefore attributed to the stretching, bending and rocking modes, respectively, of the Si-O-Si bridges. The shift of the Si-O-Si stretching band observed at 1044 cm⁻¹ for Type I coatings with respect to the calculated value of 1100 cm⁻¹ suggests the coating to be less dense (Pecheur et al., J. Non-Cryst. Solids 245 (1999) 20). The absorption bands in the region 1127 - 1226 cm⁻¹ are ascribed to SiO₂ phonon modes (Hu, J. Appl. Phys. 51 (1980) 5945). In addition, several hydrocarbon related bonds are also observed in Figure 9. The C=O stretching band at 1712 cm⁻¹, the symmetric and asymmetric C-H bending of -CH₃ groups at 1419 and 1472 cm⁻¹, respectively and C-H stretches around 2875, 2930 and 2960 cm⁻¹ are typical of C-H in ethoxy groups (Tedder et al., J. Appl. Phys. 69 (1991) 7037). As a result of the presence of hydrocarbon in the coatings (described in Section 2.3.2 below), the most intense absorption band around 1044 cm⁻¹ corresponding to the Si-O-Si structure may partly be due to Si-O-C groups and the absorption band at 796 cm⁻¹ may also be attributed to Si-O-CₓH_{y}.

### 2.3.2 XPS studies

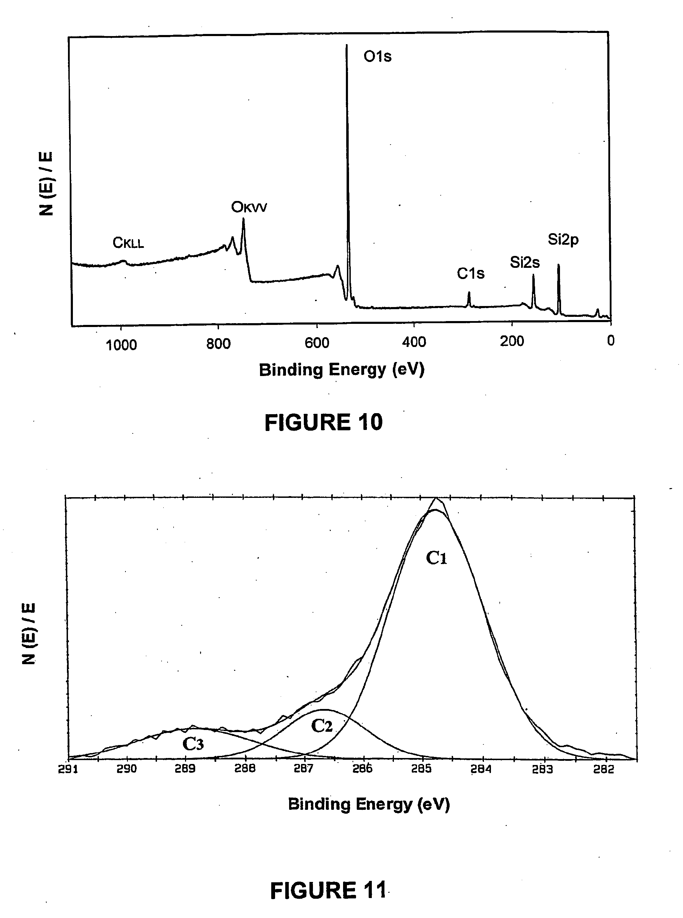

Figure 10 shows a wide-scan XPS spectrum of a Type I coating and illustrates the presence of silicon, oxygen and carbon elements in the coating. No titanium signal from the substrate is observed. The C1s photoelectron peak is broad and asymmetrical. A deconvolution of this peak appears to contain at least three peaks (Figure 11). The C1s peak at 284.8 eV corresponds to saturated hydrocarbon C-H, and the other peaks (C2, C3) at 286.6 and 288.8 eV are attributed to C-O (from ethoxy group) and C=O or O-C-O bonds, respectively. The BE values of Si2p and 01s photoelectron peaks were observed at 103.1 eV and 532.5 eV, respectively. These values are close to those reported for silica gel (103.6 eV for Si2p and 532.4 eV for 01s) (Vallee et, al., J. Vac. Sci. Technol. A 18 (2000) 2728). The shape and position of the Si2p peak is typical of SiO₂-like films, suggesting that Si is bonded to four oxygen atoms. However, the O/Si ratio in Type I coatings, as determined by quantitative XPS, was observed to be 2.3, a value larger than the stoichiometric value of 2.0 for SiO₂. This type of increase in the O/Si ratio can be explained on the basis of increased Si-O-H and Si-O-CₓH_{y} types of bonds at the cost of Si-O-Si bonds.

The above FTIR and XPS observations suggest that organic groups (such as ethoxy) are incorporated into Type I coatings. The most likely source of these groups is the TEOS fragments such as di-and tri-ethoxysilane present in the plasma reactor during deposition.

### 2.3.3 Interaction with SBF

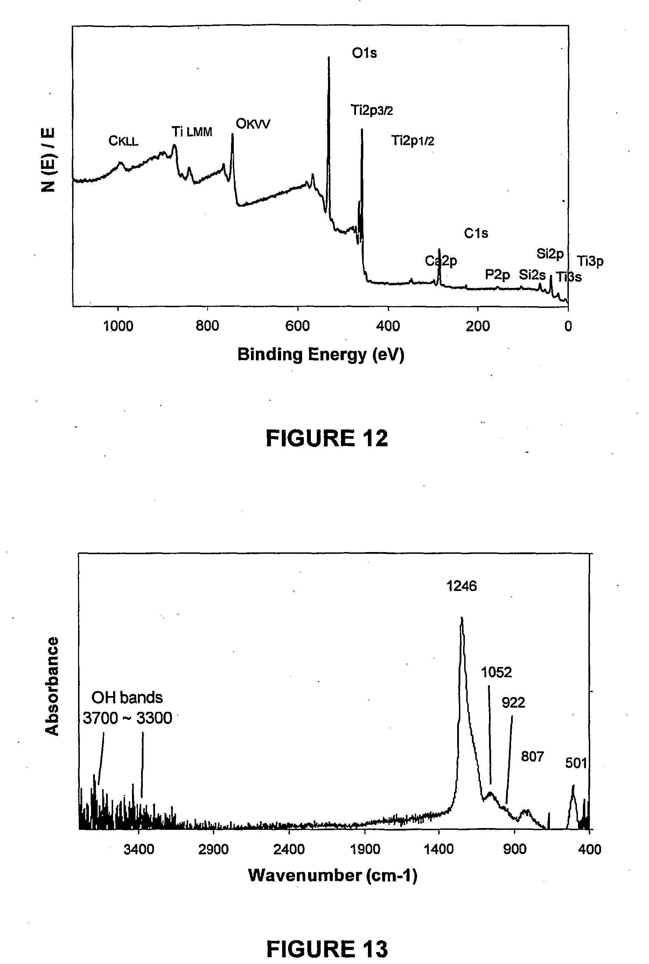

To study the interaction of the hydroxylated silica coating with SBF, the coating was immersed in 40 ml of the standard SBF at 37°C for fourteen days. XPS wide-scan of this sample shown in Figure 12 contains only very small surface concentrations of calcium and phosphate. The Si2p photoelectron peak was reduced in its intensity and a strong titanium peak reappeared. SEM micrographs after SBF reaction showed no evidence of new morphology and were similar to those of the original substrate surfaces.

Since the XPS spectrum of the PACVD coating (Figure 10) did not show any trace of titanium but a strong silicon signal on the coating surface, it is therefore clear that the silica coating had largely detached or dissolved into the SBF solution. This observation showed that the adhesive quality of the Type I coatings was poor in its reactivity with SBF. There may be two reasons for this behaviour. One is the lack of reaction between the metal substrate and the coating, i.e., limited development of a graded composite structure. The second reason is the excessive incorporation of OH and organic (hydrocarbon) groups within the silica coating structure, making the coatings unstable through hydrolysis and dissolution in the SBF solution. The FTIR spectrum of Figure 9 shows a high concentration of OH groups within the coating. This technique analyses the bulk of the coating with analysis depth >0.7 µm in specular reflectance for the 3000 - 4000 cm⁻¹ region. Hydroxyl groups being a product of the TEOS reaction with water can be incorporated into the silica coatings. The incorporation of organic (ethoxy) groups in Type I coatings is revealed by the FTIR and XPS measurements mentioned above. It is the incorporation of the Si-OH, C-H and CO groups into the coating that causes the SiO₂ network to break down, leading to low-density coatings. These coatings containing OH and organic groups provide access and sites for water absorption. Hence hydrolysis of the coating occurs and eventually disintegrates into the SBF solution. These characteristics appear to be a consequence of the high TEOS/air ratio and rapid deposition of silica when compared with our earlier results.

It follows from the above discussion that the incorporation of hydroxyl and organic groups should be eliminated or minimised from the bulk of the silica coatings in view of their instability in SBF. A mechanism for the removal of organic groups (mainly ethoxy) from the depositing silica coatings has been proposed in which the organic groups are etched away by the oxygen species present in an oxygen-rich TEOS plasma. In view of our own mass spectrometry measurements (Arora et al., Surf. Interface Anal. 24 (1996) 539) performed at low deposition ratio on the TEOS/water plasma, we believe that the ethoxy groups present in the plasma combine with active hydrogen species in gas phase to form CH₃CH₂OH which is then pumped away; i.e. they are not incorporated into the growing film. If the ratio of active oxygen in the plasma at the surface of the growing coating is high enough, hydrocarbon incorporation into the coating will be significantly reduced or even prevented. To the contrary, if there are not enough active oxygen atoms to fully remove the ethoxy groups and their undesirable fragments absorbed on the growing coating surface (i.e. high TEOS/air ratio as in Type I), some organic groups and silanol groups will be incorporated into the deposited coatings. The observation in FTIR spectra of Si-O-C vibrations suggests that there is extensive reaction of the hydrocarbon fragments with silica during the deposition inhibiting the formation of the composite structure in the oxide layer.

The removal of OH groups from the depositing silanol (Si-OH) species in the silica coatings has also been observed in an oxygen-rich TEOS plasma. This observation has also been attributed to the low deposition rate under this condition wherein two depositing Si-OH species react to eliminate a water molecule and deposit silica as a result.

The preceding results suggest that oxygen-rich TEOS plasma should be used for the deposition of adhesive silica coatings stable in SBF for growth of calcium hydroxy phosphate. Therefore, we prepared and investigated silica coatings onto titanium substrates using low TEOS/air ratio (air-rich TEOS) plasma. The results obtained on these coatings (Type II) are described below.

### 2.4 - Coatings deposited using low TEOS/air ratio (Type II coatings)

The low TEOS/air ratio used for depositing these coatings was achieved by reducing the TEOS flow into the deposition chamber. As described in Example 2.3, this was carried out by lowering the temperature of the TEOS-containing bulb to 8°C. As a result of the reduced TEOS/air ratio, the deposition rate of the coating was also reduced.

### 2.4.1 Infrared studies

An infrared spectrum of a Type II silica coating is shown in Figure 13. The peak near 1052 cm⁻¹ is attributed to the stretching mode of the Si-O-Si group, with its symmetric stretch centred at about 501 cm⁻¹. The absorbance due to the Si-O species is observed at -807 cm⁻¹. The absorption band at 1246 cm⁻¹ is ascribed to the SiO₂ phonon mode. The weak absorbance signal at 922 cm⁻¹ corresponds to the Si-OH stretching vibration, and the weak absorbance bands with very low signal to noise ratio observed around 3650 and 3400 cm⁻¹ are due to the SiO-H stretch. A comparison of the 3000 - 3700 cm⁻¹ region of IR spectra of Type II coatings (Figure 13) with those of Type I coatings (Figure 9) clearly suggest that only a small amount of the Si-OH species is present in Type II coatings. The near-absence of C-H absorption bands in Figure 13 also suggests that virtually no hydrocarbon species of the TEOS reaction products get incorporated into Type II coatings.

### 2.4.2 XPS studies

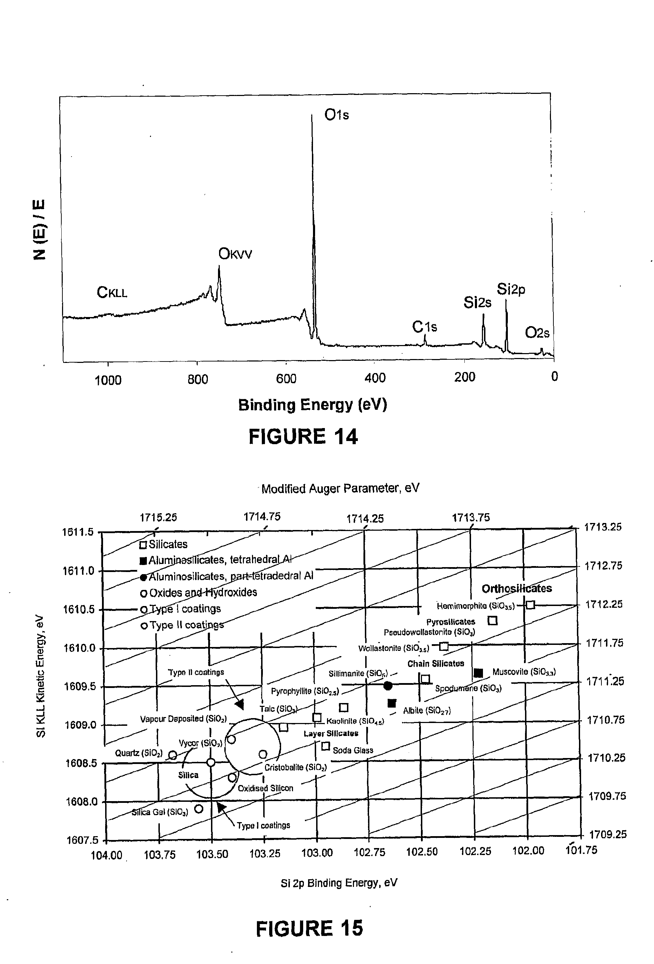

Figure 14 shows the XPS spectrum of a Type II silica coating surface (2 - 5 nm analysis depth). The absence of titanium signal in this wide-scan XPS spectrum shows that the coating has fully covered the titanium metal substrate. The coating is stoichiometric with the O/Si ratio of 2.0 (Table 4). The binding energy values of the Si2p and O1s peaks are observed to be 103.2 and 532.4 eV, respectively. These values are close to the binding energy values commonly obtained for silica gel (103.6 and 532.4 eV). The C1s photoelectron intensity is smaller than that observed for Type I coatings, suggesting reduced hydrocarbon contamination of Type II coating surfaces.

**Table 4. Atomic concentration (%) of the two types of hydroxylated silica coatings.**

| Element | Type I | Type II |
|---|---|---|
| C | 11.4 | 7.9 |
| Si | 27.1 | 30.7 |
| O | 61.5 | 61.4 |
| O/Si | 2.3 | 2.0 |

From the XPS, the modified Auger parameter (defined as the sum of the kinetic energy of the most intense Auger transition and the binding energy of the most intense core photoionization for a given element) was used to compare the chemical nature of our silica coatings with other types of silica (Arora et al., Surf. Interface Anal. 24 (1996) 539 and Wagner et al., J. Vac. Sci. Technol. 21 (1982) 933). Table 5 lists the values of the binding energy of the Si2p photoelectron and the kinetic energy of the Si KKL Auger electron observed for both Type I and Type II silica coatings. The modified Auger parameter values obtained using the data of Table 5 are plotted in Figure 15 along with some of the values compiled for different silicates (Wagner et al., J. Vac. Sci. Technol. 7 (1996) 59). The figure exhibits the Si2p binding energy on the x-axis, the Si KLL Auger kinetic energy on the left y-axis and the modified Auger parameter on the right y-axis. This plot presents a transition from ortho- and di-silicate structures in the top right-hand corner through layer silicates to bulk silica structures in the lower left-hand corner. The positions of the modified Auger parameter values obtained for our silica coatings in the plot (Figure 15) give modified Auger parameter values intermediate between vapour deposited silica or quartz with relatively low SiOH coverage, and silica gel with extensive hydroxylation. The Si2p and SiKLL values show that the Type I coatings exhibit a structure close to a silica gel whereas a structure closer to a vapour deposited silica with partial surface hydroxylation is exhibited by the Type II coatings.

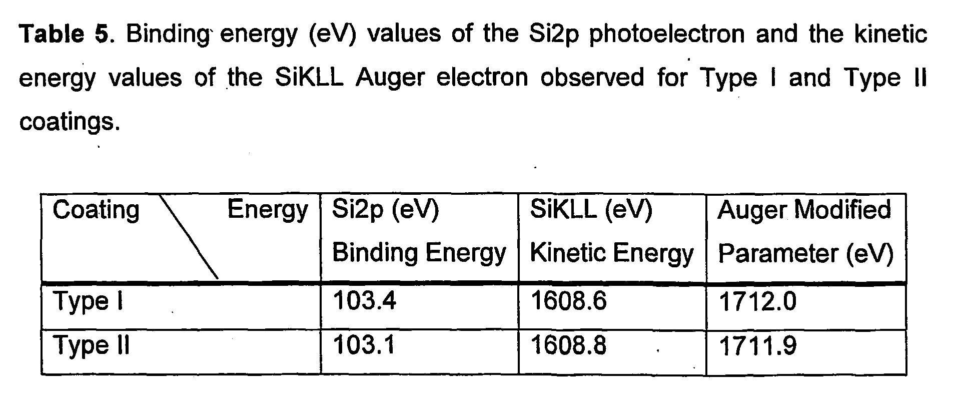

### 2.4.3 Interaction with SBF

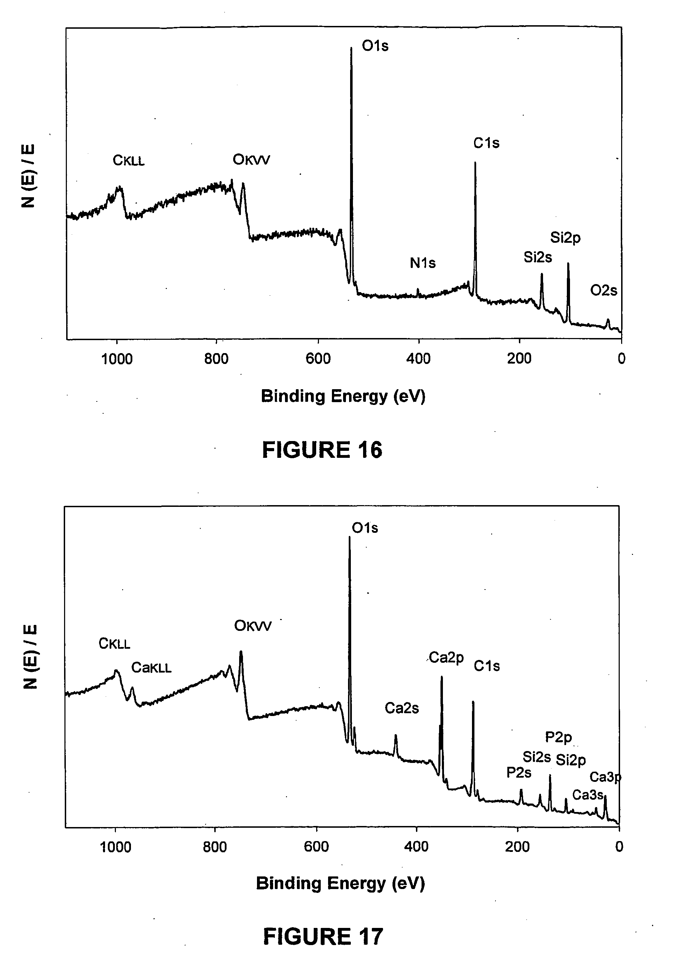

The structural and compositional results presented above suggest that Type II coatings should exhibit better chemical stability compared to Type I coatings, in particular in SBF. To study the interaction of Type II coatings with SBF, one such sample was immersed in 40 ml of the SBF with amine stabilization at 37°C for a period of fourteen days. The XPS analysis of the sample retrieved after this SBF immersion test revealed the presence of the silica coating intact on the titanium substrate. No titanium signal from the substrate was observed (Figure 16; Table 6). There still were O and Si present on the surface and the O/Si ratio was retained close to 2.0. These observations show that Type II silica coatings are stable in the SBF. The XPS spectrum also revealed that in addition to the increased C1s signal, a weak N1 s signal was present on the surface of the coating consistent with absorbed amine. However, there were no photoelectron signals from either Ca or P, the elements expected to have deposited onto the coating in the form of calcium hydroxy phosphate from the SBF. The pH-stabilized SBF has tris-hydroxymethyl aminomethane [(CH₂OH)₃CNH₂] as a buffering agent which appears to have absorbed onto the silica coating, thus blocking the deposition of Ca²⁺ and PO₄³⁻ ions onto the silica surface.

It appears that the surface chemistry of the PACVD. - hydroxylated silica coating is different from that of a hydroxylated silica gel, which is reported to induce calcium hydroxy phosphate precipitation on its surface when treated with amine-containing SBF. In our case, the amine is preferentially absorbed. It has been suggested (Pereira et al., J. Am. Ceram. Soc. 78 (1995) 2463 and Pereira et al., J. Sol-Gel Sci. Technol. 7 (1996) 59) that on the hydroxylated silica gel surface, the negatively charged pore volume and pore size provide favourable conditions for the nucleation of calcium hydroxy phosphate and its subsequent growth and these conditions may not apply in our reacted coatings.

Since the growth of calcium hydroxy phosphate on PACVD silica coatings was one of our objectives, the interaction of Type II coatings with amine-free SBF (without the buffering agent tris-hydroxymethyl aminomethane) was also studied. For this, a fresh SBF solution was prepared without tris-hydroxymethyl aminomethane and Type II samples were immersed in it at 37°C for a period of fourteen days. The XPS spectrum of a retrieved sample (Figure 17; Table 6) revealed the presence of Ca, P, C and O signals on the sample surface. The binding energy values of the Ca2p and P2p photoelectrons were determined to be 347.0 and 133.0 eV, respectively. These values match those observed for standard, sintered hydroxyapatite (Takadama et al., J. Biomed. Mater. Res. 55 (2001) 185). The absence of titanium and the presence of Si in the XPS spectrum imply that Type II silica coatings when treated with anime-free SBF retain their chemical stability and at the same time grow a thin layer containing calcium and phosphate on them. The observation of Si signals from the Type II layer after SBF reaction suggests that the deposited layer is <10nm thick at least in some areas.

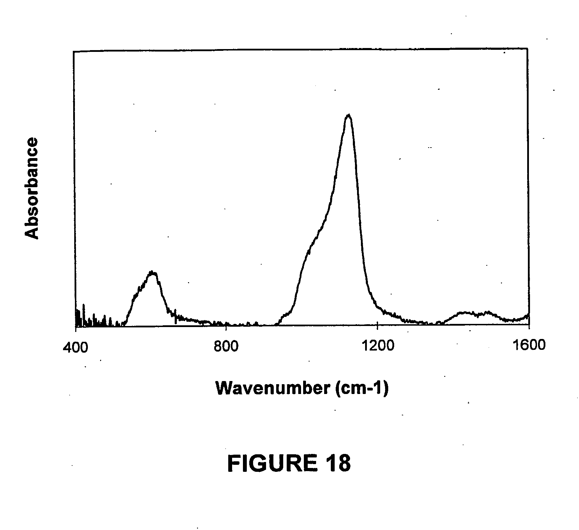

The layer thus formed is confirmed to be calcium hydroxy phosphate, as revealed by its FTIR absorption characteristics shown in Figure 18. The two peaks observed in the regions 950 - 1200 cm⁻¹ and 500 - 650 cm⁻¹ are attributed to the major absorption modes associated with the presence of phosphate (Russell et al., J. Am. Ceram. Soc. 79 (1996) 837). The weak peak in the region 1400 - 1500 cm⁻¹ is attributed to a carbonate group (CO₃²⁻), indicating the incorporation of carbonate into the apatite lattice as found by other researchers and in accordance with *in vivo* growth.

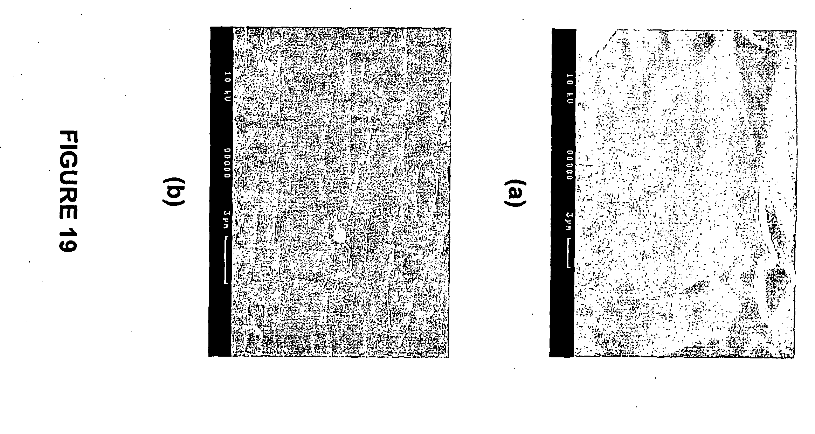

The surface morphology of this calcium hydroxy apatite layer, as revealed by SEM, is shown in Figure 19 b. For comparison, the surface morphology of a Type II silica coating before SBF reaction is shown in Figure 19 a. The apatite layer appears coherent and conformal on the surface with some regions of ridged and blocky structure.

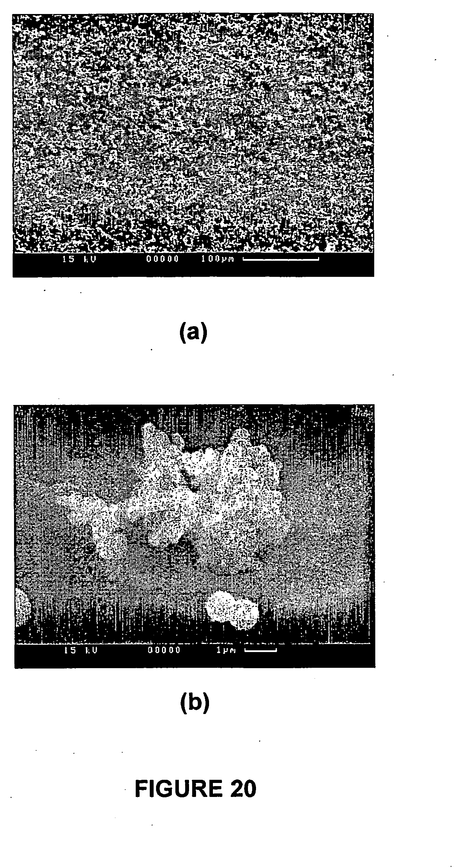

Reaction in higher SBF super-saturation (i.e. 1.5SBF for 5 days) reveals another interesting result potentially relevant to the structure of the final HA films. Figures 20 a and b show that, at this higher super-saturation, precipitated aggregates with a variety of morphologies are attached to the surface. These aggregates overly the continuous, coherent layer formed at 1.0SBF (Fig. 19b). The aggregates are both spheroidal and blocky with porosity on the 0.1 - 1µm scale. They appear to individually comprise much smaller crystallites at the same scale as those in the underlying continuous film (i.e. ∼0.1µm). Their appearance only at higher super-saturation suggests that they are formed by precipitation in solution and then absorbed on to the pre-formed continuous calcium hydroxy phosphate layer.

**Table 6. XPS atomic concentrations (%) of Type II coatings treated with SBF and amine-free SBF.**

| Element | Type II coatings soaked in SBF (with amine) for 14 days | Type II coatings soaked in SBF (without amine) for 14 days |
|---|---|---|
| C | 47.4 | 39.0 |
| Si | 16.9 | 4.1 |
| O | 34.2 | 39.3 |
| N | 1.5 | 0.0 |
| Ca | 0.0 | 9.1 |
| P | 0.0 | 8.5 |

The growth of calcium hydroxy phosphate onto PACVD-deposited hydroxylated silica coatings *via* the interaction with simulated body fluid (SBF) has been studied. Two types of coatings were identified. Type I silica coatings deposited using high TEOS/air ratio and rapid deposition have been shown to contain high concentrations of hydrocarbon and silanol groups. The excessive incorporation of these groups into the bulk of Type I coatings make them unstable through using low TEOS/air ratio, the slower deposition rate and the presence of sufficient oxygen in the depositing plasma help to efficiently eliminate the incorporation of hydrocarbon and silanol groups into the bulk of the coatings.

Only the surface of the coating was hydroxylated by the H₂O₂ plasma. Type II coatings are stable in SBF. The modified Auger parameter values obtained for our silica coatings suggest that Type I coatings exhibit a structure closer to a silica gel whereas a structure closer to a vapour deposited silica partially hydroxylated is exhibited by Type II coatings. Owing to the chemical stability of Type II coatings in SBF, the growth of calcium hydroxy phosphate from amine-free SBF can be realized on these coatings. The presence of amine stabiliser in SBF, however, inhibits the growth of calcium hydroxy phosphate on Type II coatings. At higher super-saturation, aggregate structures appear overlying the continuous calcium hydroxy phosphate layer.

## Claims

1. A method of forming a graded composite silica bioactive coating on a metallic biomedical implant, the method including the step of contacting at least part of the surface of the implant prior to implantation with a plasma gas containing a reactive hydroxylating oxidant species,
preceded by a step of exposing the implant to an organosilane or organosilicate species to form a silica coating on at least part of the implant prior to contact with the oxidant species, wherein the step of exposing the implant to an organosilane or organosilicate species includes exposing the implant to a plasma gas formed from the organosilane or organosilicate containing air or oxygen.

2. A method as in claim 1, wherein the reactive hydroxylating oxidant species that is included in the plasma gas is formed using an agent selected from the list consisting of hydrogen peroxide, water, oxygen/water and air/water.

3. A method as in claim 2, wherein the agent that is used to form the reactive hydroxylating oxidant species in the plasma gas is hydrogen peroxide.

4. A method as in any one of the preceding claims, wherein the implant is made from stainless steel, titanium, titanium alloy, or cobalt-chromium.

5. A method as in claim 4, wherein the implant is made from stainless steel.

6. A method as in any one of the preceding claims, wherein the organosilane is a tetralkylorganosilane containing alkyl groups having between 1 and 10 carbon atoms.

7. A method as in claim 6, wherein the organosilane is a tetralkylorganosilane containing alkyl groups having between 1 and 5 carbon atoms.

8. A method as in claim 7, wherein the organosilane is tetraethoxysilane.

9. A method as in claim 1, wherein the ratio of organosilane or organosilicate to air or oxygen is varied over time to deposit a graded composite silica coating.

10. A biomedical implant having a bioactive coating that is formed using the method of any one of the preceding claims.

11. A bioactive biomedical implant as in claim 10, wherein the implant has a hydroxylated, graded composite silica coating having an average thickness of less than 300 nm.

12. A bioactive biomedical implant as in claim 10, wherein the implant has a hydroxylated, graded composite silica coating having an average thickness of less than 200 nm.

13. A bioactive biomedical implant as in claim 10, wherein the implant has a hydroxylated, graded composite silica coating having an average thickness of less than 100 nm.

## Patentansprüche

1. Verfahren zum Bilden einer abgestuften zusammengesetzten bioaktiven Siliciumdioxid-Beschichtung auf einem metallischen biomedizinischen Implantat, wobei das Verfahren den Schritt umfasst, in dem zumindest ein Teil der Oberfläche des Implantats vor der Implantation mit einem Plasmagas in Kontakt gebracht wird, das eine reaktive hydroxylierende Oxidationsspezies enthält,
dem ein Schritt vorausgeht, in dem das Implantat einer Organosilan- oder Organosilikat-Spezies ausgesetzt wird, um eine Siliciumdioxid-Beschichtung auf zumindest einem Teil des Implantats vor dem Kontakt mit der Oxidationsspezies zu bilden, wobei der Schritt, in dem das Implantat einer Organosilan- oder Organosilikat-Spezies ausgesetzt wird, umfasst, das Implantat einem Plasmagas auszusetzen, das aus Luft oder Sauerstoff, die bzw. der Organosilan oder Organosilikat enthält, gebildet ist.

2. Verfahren nach Anspruch 1, wobei die reaktive hydroxylierende Oxidationsspezies, die im Plasmagas enthalten ist, unter Verwendung eines Mittels gebildet wird, das aus der Liste ausgewählt ist, die aus Wasserstoffperoxid, Wasser, Sauerstoff/Wasser und Luft/Wasser besteht.

3. Verfahren nach Anspruch 2, wobei das Mittel, das verwendet wird, um die reaktive hydroxylierende Oxidationsspezies im Plasmagas zu bilden, Wasserstoffperoxid ist.

4. Verfahren nach einem der vorangehenden Ansprüche, wobei das Implantat aus Edelstahl, Titan, Titanlegierung oder Kobalt-Chrom besteht.

5. Verfahren nach Anspruch 4, wobei das Implantat aus Edelstahl besteht.

6. Verfahren nach einem der vorangehenden Ansprüche, wobei das Organosilan ein Tetraalkylorganosilan, das Alkylgruppen mit zwischen 1 und 10 Kohlenstoffatomen enthält, ist.

7. Verfahren nach Anspruch 6, wobei das Organosilan ein Tetraalkylorganosilan, das Alkylgruppen mit zwischen 1 und 5 Kohlenstoffatomen enthält, ist.

8. Verfahren nach Anspruch 7, wobei das Organosilan Tetraethoxysilan ist.

9. Verfahren nach Anspruch 1, wobei das Verhältnis von Organosilan oder Organosilikat zu Luft oder Sauerstoff über die Zeit verändert wird, um eine abgestufte zusammengesetzte Siliciumdioxid-Beschichtung abzuscheiden.

10. Biomedizinisches Implantat mit einer bioaktiven Beschichtung, die unter Verwendung des Verfahrens nach einem der vorangehenden Ansprüche gebildet wird.

11. Bioaktives biomedizinisches Implantat nach Anspruch 10, wobei das Implantat eine hydroxylierte, abgestufte zusammengesetzte Siliciumdioxid-Beschichtung mit einer mittleren Dicke von weniger als 300 nm aufweist.

12. Bioaktives biomedizinisches Implantat nach Anspruch 10, wobei das Implantat eine hydroxylierte, abgestufte zusammengesetzte Siliciumdioxid-Beschichtung mit einer mittleren Dicke von weniger als 200 nm aufweist.

13. Bioaktives biomedizinisches Implantat nach Anspruch 10, wobei das Implantat eine hydroxylierte, abgestufte zusammengesetzte Siliciumdioxid-Beschichtung mit einer mittleren Dicke von weniger als 100 nm aufweist.

## Revendications

1. Procédé de formation d'un revêtement bioactif en silice composite graduée sur un implant biomédical métallique, le procédé incluant l'étape consistant à mettre en contact au moins une partie de la surface de l'implant avant l'implantation avec un gaz plasma contenant une espèce d'oxydant d'hydroxylation réactif,
précédée d'une étape consistant à exposer l'implant à une espèce d'organosilane ou d'organosilicate afin de former un revêtement en silice sur au moins une partie de l'implant avant de le mettre en contact avec l'espèce d'oxydant, dans lequel l'étape consistant à exposer l'implant à une espèce d'organosilane ou d'organosilicate inclut d'exposer l'implant à un gaz plasma formé à partir de l'organosilane ou de l'organosilicate contenant de l'air ou de l'oxygène.

2. Procédé selon la revendication 1, dans lequel l'espèce d'oxydant d'hydroxylation réactif qui est incluse dans le gaz plasma est formée en utilisant un agent sélectionné à partir de la liste constituée de peroxyde d'hydrogène, d'eau, d'oxygène/d'eau et d'air/d'eau.

3. Procédé selon la revendication 2, dans lequel l'agent qui est utilisé pour former l'espèce d'oxydant d'hydroxylation réactif dans le gaz plasma est du peroxyde d'hydrogène.

4. Procédé selon l'une quelconque des revendications précédentes, dans lequel l'implant est constitué d'acier inoxydable, de titane, d'un alliage de titane, ou de cobalt-chrome.

5. Procédé selon la revendication 4, dans lequel l'implant est constitué d'acier inoxydable.

6. Procédé selon l'une quelconque des revendications précédentes, dans lequel l'organosilane est un tétralkyl-organosilane contenant des groupes alkyles ayant entre 1 et 10 atomes de carbone.

7. Procédé selon la revendication 6, dans lequel l'organosilane est un tétralkyl-organosilane contenant des groupes alkyles ayant entre 1 et 5 atomes de carbone.

8. Procédé selon la revendication 7, dans lequel l'organosilane est un tétraéthoxysilane.

9. Procédé selon la revendication 1, dans lequel le rapport de l'organosilane ou de l'organosilicate sur l'air ou l'oxygène varie au fil du temps pour déposer un revêtement en silice composite graduée.

10. Implant médical ayant un revêtement bioactif qui est formé en utilisant le procédé selon l'une quelconque des revendications précédentes.

11. Implant biomédical bioactif selon la revendication 10, dans lequel l'implant a un revêtement en silice composite graduée, d'hydroxylation ayant une épaisseur moyenne inférieure à 300 nm.

12. Implant biomédical bioactif selon la revendication 10, dans lequel l'implant a un revêtement en silice composite graduée, d'hydroxylation ayant une épaisseur moyenne inférieure à 200 nm.

13. Implant biomédical bioactif selon la revendication 10, dans lequel l'implant a un revêtement en silice composite graduée, d'hydroxylation ayant une épaisseur moyenne inférieure à 100 nm.
